**Europäisches Patentamt**

**European Patent Office**

**Office européen des brevets**

(11) Veröffentlichungsnummer: **0 426 083 A1**

# EUROPÄISCHE PATENTANMELDUNG

(21) Anmeldenummer: **90120730.8**

(22) Anmeldetag: **29.10.90**

(51) Int. Cl.⁵: **G01N 33/68**, G01N 33/573, G01N 33/566

(30) Priorität: **31.10.89 DE 3936256**

(43) Veröffentlichungstag der Anmeldung:
**08.05.91 Patentblatt 91/19**

(84) Benannte Vertragsstaaten:
**AT BE CH DE DK ES FR GB GR IT LI LU NL SE**

(71) Anmelder: **Max-Planck-Gesellschaft zur Förderung der Wissenschaften e.V.
Bunsenstrasse 10
W-3400 Göttingen(DE)**

(72) Erfinder: **Justus, Klaus W., Dr.
Gaffkystrasse 11
W-6300 Giessen(DE)**
Erfinder: **Kramer, Michael, Dr.
Bergstrasse 85
W-6102 Pfungstadt(DE)**

(74) Vertreter: **Huber, Bernhard, Dipl.-Chem. et al
Patentanwälte H. Weickmann, Dr. K. Fincke
F.A. Weickmann, B. Huber Dr. H. Liska, Dr. J.
Prechtel Möhlstrasse 22 Postfach 860 820
W-8000 München 86(DE)**

(54) **Test zur differentiellen Quantifizierung des freien bzw. des peptidasekomplexierten Proteinaseinhibitors alpha2-Makroglobulin.**

(57) Zur spezifischen Bestimmung der nativen Form oder der komplexierten Form von $\alpha_2$-Makroglobulin inkubiert man die Lösung mit einem für die nachzuweisende $\alpha_2$-Makroglobulinform spezifischen immobilisierten Rezeptor und einem mit $\alpha_2$-Makroglobulin bindefähigen markierten Rezeptor, wobei man als spezifische Rezeptoren diejenigen der Streptokokken-Spezies Stamm 2 (DSM 5532), Stamm 3 (DSM 5533) oder von Streptokokkus spp Stamm 1 (DSM 5531) verwendet, trennt feste von flüssiger Phase und mißt nach Trennung der Phasen die Markierung in einer der beiden Phasen.

EP 0 426 083 A1

## TEST ZUR DIFFERENTIELLEN QUANTIFIZIERUNG DES FREIEN BZW. DES PEPTIDASEKOMPLEXIERTEN PROTEINASEINHIBITORS $\alpha_2$-MAKROGLOBULIN

Die Erfindung betrifft ein Verfahren zum spezifischen Nachweis der nativen Form oder der komplexierten Form von $\alpha_2$-Makroglobulin.

$\alpha_2$-Makroglobulin (im folgenden als $\alpha_2$M) bezeichnet, ist ein Proteaseinhibitor mit breitem Wirkungsspektrum. Eukaryontische und prokaryontische Proteasen aller funktionellen Klassen, d.h. Serin-, Zystein-, Aspartat- und Metallo-Proteasen werden von $\alpha_2$M inhibiert (Van Leuven, F. (1982) Trends Biochem.Sci 7: 185-187; Travis J., Salvesen GS (1983) Ann.Rev. Biochem. 52: 655-680). In humanem Plasma liegt $\alpha_2$M in einer Konzentration von ungefähr 2 g/l vor. Es ist jedoch bekannt, daß ungefähr 40 % des gesamten im Körper vorhandenen $\alpha_2$M im extravaskulären Raum vorliegen und langsam mit dem vaskulären $\alpha_2$M ausgetauscht werden (Saksela O. (1985) Biochim.Biophys. Acta 823: 35-65). $\alpha_2$M ist in nativer Form ein Glykoprotein mit einem Molekulargewicht von ungefähr 700 kDa und besteht aus vier identischen Untereinheiten mit einem apparenten Molekulargewicht von 170 kDA.

Der inhibitorische Reaktionsmechanismus von $\alpha_2$M wird durch die sogenannte "Trap"-Hypothese erklärt (Barret A. J., Starkey P.M. (1973) Biochem.J. 133: 709-724). Danach führt die Interaktion einer Peptidase zur Spaltung einer Peptidbindung innerhalb des $\alpha_2$M-Moleküls im Bereich der sog. "bait region". Diese Spaltung führt zu einer Konformationsänderung im Sinne einer Kondensierung bzw. Straffung des Enzym-Inhibitor-Komplexes. Diese Konformationsänderung kann gelelektrophoretisch nachgewiesen werden, da bei der elektrophoretischen Trennung die native Form langsamer wandert und daher als "slow form" bzw. $\alpha_2$MS bezeichnet wird, während die komplexierte Form schneller wandert und daher als "fast form" oder $\alpha_2$MF bezeichnet wird. Die molekulare Umwandlung von $\alpha_2$M in die komplexierte Form kann auch durch nicht enzymatische Mechanismen wie z.B. die Inkubation mit niedermolekularen Methylammoniumverbindungen induziert werden.

Nach dem Einfangen der Proteinase bleibt das aktive Zentrum des Enzyms unbeeinflußt, so daß niedrigmolekulare Substrate noch umgesetzt werden können, während der Abbau höhermolekularer Substrate sterisch behindert wird.

Aufgrund der breiten inhibitorischen Aktivität von $\alpha_2$M und seiner Gegenwart im extravaskulären und interstitiellen Raum wird angenommen, daß $\alpha_2$M extrazelluläre proteolytische Vorgänge kontrolliert. Es wird vermutet, daß $\alpha_2$M im Überschuß auftretende, potentiell pathogene Peptidasen dann inhibiert, wenn die jeweiligen, spezifischen Inhibitorsysteme erschöpft sind. Der spezifische Nachweis der beiden unterschiedlichen Formen von $\alpha_2$M könnte daher ein wertvoller diagnostischer Parameter sein.

Bisher konnten die beiden unterschiedlichen Formen von $\alpha_2$M in Körperflüssigkeiten nur durch arbeits- und kostenintensive biochemische Verfahren sicher unterschieden werden, die für breit angelegte Reihenuntersuchungen an Probenmaterial von Patienten und Normalpersonen nicht geeignet sind. Eine $\alpha_2$M Nachweismethode gemäß CA Vol 107 (1987), 757009 unter Verwendung von monoklonalen Antikörpern eignet sich icht für diese Unterscheidung.

Es war daher Aufgabe der Erfindung, ein Verfahren zur Verfügung zu stellen, das eine quantitative Unterscheidung der beiden Formen von $\alpha_2$M in biologischen Flüssigkeiten ermöglicht und schnell und einfach durchzuführen ist. Darüber hinaus war es Aufgabe der Erfindung, eine Möglichkeit zu schaffen, um in $\alpha_2$M komplexierte Peptidasen spezifisch nachweisen zu können.

Diese Aufgabe wird gelöst durch ein Verfahren zur spezifischen Bestimmung der nativen Form oder der komplexierten Form von $\alpha_2$-Makroglobulin, welches dadurch gekennzeichnet ist, daß man die Lösung mit einem für die nachzuweisende $\alpha_2$-Makroglobulinform spezifischen immobilisierten Rezeptor und einem mit $\alpha_2$-Makroglobulin bindefähigen markierten Rezeptor inkubiert, wobei man als spezifische Rezeptoren diejenigen der Streptokokken-Spezies Stamm 2 (DSM 5532), Stamm 3 (DSM 5533) oder von Streptokokkus spp Stamm 1 (DSM 5531) verwendet, feste von flüssiger Phase trennt und nach Trennung der Phasen die Markierung in einer der beiden Phasen mißt.

Das erfindungsgemäße Verfahren ermöglicht es, die native Form bzw. die komplexierte Form von $\alpha_2$M spezifisch in Körperflüssigkeiten nachzuweisen. Darüber hinaus gelingt es in einfacher Weise, die in der komplexierten Form von $\alpha_2$M gebundene Protease nach Art und Konzentration zu bestimmen. Die differentielle quantitative Bestimmung der beiden $\alpha_2$M-Formen erfolgt erfindungsgemäß durch ein Verfahren nach dem Prinzip des Immunoassay. Dazu wird die Körperflüssigkeit mit zwei Rezeptoren inkubiert, von denen der eine an eine feste Phase gebunden ist und der andere eine Markierung trägt. Bei der Inkubation bilden sich Komplexe aus der nachzuweisenden Form des $\alpha_2$M und dem markierten Antikörper, die an die Festphase über den dort immobilisierten Rezeptor gebunden werden. Die freie Form des markierten Rezeptors wird von der gebundenen Form durch Trennung der festen von der flüssigen Phase isoliert.

Anschließend wird die Markierung in einer der beiden Phasen gemessen und ist ein Maß für die Menge der jeweils zu bestimmenden Form von $\alpha_2$M.

Als festphasengebundener Rezeptor zur Bestimmung von freiem $\alpha_2$M wird der Rezeptor von Streptokokkus spp. Stamm 2, DSM 5532 bzw. Stamm 3, DSM 5533 verwendet, während zur Bestimmung von komplexiertem $\alpha_2$M von Streptokokkus spp. Stamm 1, DSM 5531 produzierte Rezeptoren verwendet werden.

Die gewonnenen Rezeptoren werden in üblicher Weise an eine feste Phase gebunden. Verfahren hierzu sind dem Fachmann bekannt und bedürfen keiner näheren Erläuterung. Als feste Phase geeignet sind die an sich bekannten Materialien, die in Form von Reaktionsröhrchen, Mikrotiterplatten, Kugeln oder Streifen vorliegen können.

Für den bindefähigen markierten Rezeptor wird ein Rezeptor verwendet, der mit $\alpha_2$-Makroglobulin bindefähig ist. Hier sind alle Rezeptoren geeignet, die mit $\alpha_2$M eine Bindung eingehen. Bevorzugt wird als markierter Rezeptor ein Antikörper oder Anti-körperfragment verwendet, der spezifisch für $\alpha_2$M ist. Es können sowohl polyklonale als auch monoklonale Antikörper verwendet werden, die in an sich bekannter Weise durch Immunisierung hergestellt werden. Besonders bevorzugt werden von der Zellinie ECACC M2: 89100605 produzierte monoklonale Antiköper oder deren Fragmente verwendet.

Die Markierung erfolgt in an sich bekannter Weise. Verfahren zur Markierung sind an sich bekannt. Geeignet ist die Markierung mit radioaktiven Atomen, Enzymen, Farbstoffen oder fluoreszierenden Molekülen sowie lumineszierenden Molekülen. Verfahren zum Nachweis der Markierungen sind ebenfalls bekannt und bedürfen keiner Erläuterung. Bevorzugt wird zur Markierung ein Enzym eingesetzt, insbesondere Peroxidase. Der Nachweis erfolgt dann über Substrate, die von Peroxidase unter Farbbildung umgesetzt werden.

Die Bestimmung von $\alpha_2$M kann in Körperflüssigkeiten erfolgen. Insbesondere findet sich $\alpha_2$ in Plasma oder Serum sowie in Körperflüssigkeiten und entzündlichen Exsudaten.

Der erfindungsgemäße Nachweis von $\alpha_2$M erfolgt nach dem Prinzip des Immunoassay und die hierfür bekannten Varianten können angewendet werden.

In einer Ausführungsform des erfindungsgemäßen Verfahrens wird anschließend an die Bestimmung von komplexiertem $\alpha_2$M Art und Menge der komplexierten Proteinase bestimmt. Dies geschieht nach Durchführung des Nachweises, indem man die Festphase, an der $\alpha_2$M in komplexierter Form gebunden ist, mit einem Substrat für eine Protease umsetzt. Als Substrat wird eine Verbindung verwendet, die durch Umsetzung mit der Protease ein nachweisbares Signal, insbesondere eine Farbbildung ergibt. Für Proteasen spezifische Substrate sind im Handel erhältlich. Das Substrat sollte ein Molekulargewicht von nicht mehr als 20 kDa aufweisen, da bei höhermolekularen Substanzen die Reaktion aufgrund sterischer Hinderung erschwert wird und damit zu einer Verfälschung des Ergebnisses führen kann.

Die Erfindung wird durch die folgenden Beispiele erläutert. Dabei werden folgende Abkürzungen verwendet:

- $\alpha_2$M = alpha-2-Makroglobulin
- DSM = Deutsche Sammlung von Mikroorganismen in Braunschweig
- BSA = Bovines Serum Albumin
- TW = Tween 20
- ECACC = European Collection of Animal Cell Cultures
- kDa = kilo Dalton
- $\alpha_2$MF = fast form von alpha-2-Makroglobulin
- $\alpha_2$MS = slow form von alpha-2-Makroglobulin
- A = Absorption
- PBS = Phosphat gepufferte Saline
- Ig = Immunglobulin
- ug = Mikrogramm
- w/v = Gewicht pro Volumen
- v/v = Volumen pro Volumen
- ml = Milliliter
- ng = Nanogramm
- ABTS = 2,2´-Azino-di-(3äthyl-benzthiazolinsulfonat)
- r = Korrelationskoeffizient
- Vk = Variationskoeffizient
- SD = Standardabweichung

Beispiel 1

In einer Probe wurde der Gehalt an $\alpha_2$MS bzw. $\alpha_2$MF bestimmt. Dazu wurden Mikrotiterplatten mit 20 µg/Napf mit dem entsprechenden, für das jeweilige $\alpha_2$M spezifischen Rezeptor beschichtet. Zur Bestimmung von $\alpha_2$MS wurden Bakterien von Streptokokkus spp. Species Stamm 2, DSM 5532 und Stamm 3, DSM 5533 und für die Bestimmung von $\alpha_2$MF Streptokokkus spp. Stamm Stamm 1, DSM 5531 in Todd Hewitt-Flüssigmedium kultiviert, anschließend abzentrifugiert und dreimal mit PBS gewaschen. Durch Extraktion mit 6 mol/l Guanidinhydrochlorid wurden die Rezeptoren freigesetzt. 20 µg dieses Guanidiniumhydrochloridextraktes in 100 µl 50 mM $Na_2HCO_3$, 3 mM $NaN_3$, pH 9,6 wurden pro Napf zur Beschichtung verwendet, die 18 Stunden lang bei 4° C erfolgte. Unspezifische Bindungsstellen sowie Streptokokken Ig-Rezeptoren wurden blockiert durch Zugabe von 200 µl/Napf PBS, 1 % w/v BSA (Sigma) und normales Kaninchen-Ig (1 µg/ml, isoliert aus Kaninchen-Serum durch Affinitätschromatographie über Protein-A-Sepharose-4B; Pharmacia/LKB, Freiburg, FRG) 1 Stunde lang bei 37° C.

Die vorbeschichteten Platten wurden dann zweimal mit 200 µl/Napf PBS/Tw gewaschen. Dann wurde 100 µl/Napf Probenlösung zugegeben und 1 Stunde bei Raumtemperatur inkubiert. Anschließend wurde fünfmal mit PBS/Tw gewaschen und dann 0,2 µg/ml monoklonaler, gegen $\alpha_2$M gerichteter Antikörper, verdünnt in PBS/Tw 1 % BSA und enthaltend 1 µg/ml normales Kaninchen-Ig zugegeben und eine 1 Stunde bei Raumtemperatur inkubiert. Anschließend wurde wiederum fünfmal unter Verwendung von PBS/Tw gewaschen. Die Mikrotiterplatten wurden dann inkubiert mit gegen die Fc-Region von Maus-IgG gerichteten Antikörpern, die mit Peroxidase markiert waren. Als Antikörper wurden von der Firma DIANOVA GmbH unter der Nummer 315-035-046 erhältliche Antikörper verwendet. Die Antikörper wurden 1:5000 in PBS/Tw 1 % BSA verdünnt, mit 1 µg/ml normalem Kaninchen-Ig versetzt und 1 Stunde bei Raumtemperatur inkubiert. Nach fünfmaligem Waschen mit PBS/Tw wurde dann gebundene Peroxidase nachgewiesen durch Inkubation mit 100 µl/Napf einer Lösung, die 750 µl 40 mM ABTS und 1 µl/ml $H_2O_2$ in 15 ml Substratpuffer (Natriumacetatpuffer pH 5,5) enthielt. Nach 15 Minuten wurde die Absorption bei 414 nm gemessen. Das Protokoll ist der folgenden Aufstellung zu entnehmen.

Die Ergebnisse für die Quantifizierung von gereinigtem Standard $\alpha_2$MS sind der Tabelle 1 und die Ergebnisse für $\alpha_2$MF der Tabelle 2 zu entnehmen.

| Aufbau des Testverfahrens zur Quantifizierung von $\alpha_2$ MS bzw. $\alpha_2$ MF | | |
|---|---|---|
| Schritt | Zeit | Substanzen |
| Beschichtung | über Nacht | Streptokokkenlysate (*) 100 ug/ml |
| Blockierung | 1 h | PBS-TW.([+]) 1 % BSA 10 ug Kaninchen IgG/ml |
| Proben-Inkubation | 1 h | Verdünnung in PBS-TW. 1 % BSA |
| Detektions-AK | 1 h | MAK M2 (ECACC 89100605) 1:2000 in PBS-TW. 1 % BSA |
| POD-Konjugat | 1 h | anti-Maus-Ig-POD ([++]) 1:5000 in PBS-TW. 1 % BSA |
| Enzym-Reaktion | variabel | z.B. ABTS (Messung bei 414 nm) |

(*) Für die Bestimmung der beiden verschiedenen molekularen Formen von $\alpha_2$-Makroglobulin werden Lysate von zwei verschiedenen Streptokokkenstämmen verwendet, die bei der DSM in Braunschweig bezogen werden können.
Slow-Form Stamm 2 (DSM 5532)
Stamm 3 (DSM 5533);
Fast-Form Stamm 1 (DSM 5531);
([+]) PBS-TW = Phosphatgepufferte Saline mit 0,01 % (w/v) Tween-20
([++]) Konjugat von Anti-Maus-Antikörper und Peroxidase

Durch den kombinierten Rezeptor/Antikörper Festphasentest unter Verwendung der immobilisierten Streptokokkenrezeptoren ist es möglich, $\alpha_2$MS bzw. $\alpha_2$MF getrennt in komplexen biologischen Flüssigkeiten (z.B. Plasma) zu quantifizieren. Dies wird in folgenden Tabellen verdeutlicht:

## TABELLE 1

| Quantifizierung von $\alpha_2$MS (freies $\alpha_2$M) im Rezeptor/Antikörper Festphasentest unter Verwendung von immobilisierten Streptokokkenrezeptoren (Stamm 2 [DSM 5532], Stamm 3 [DSM 5533]) | | | |
|---|---|---|---|
| ng $\alpha_2$MS/ml Probe | A 414 nm | SD | VK (%) |
| 500 | 0.957 | 0.0181 | 1.9 |
| 250 | 0.663 | 0.0174 | 2.6 |
| 125 | 0.402 | 0.0121 | 3.0 |
| 60 | 0.220 | 0.0136 | 6.1 |
| 30 | 0.174 | 0.0043 | 2.5 |
| 15 | 0.083 | 0.0081 | 9.7 |
| 500 $\alpha_2$MF/ml Probe | 0.061 | 0.0012 | 1.9 |

Für die Quantifizierung von $\alpha_2$MS ergibt sich aus Tabelle 1 eine Standardkurve mit linearer Korrelation (r = 0.982) bei einem Konzentrationsbereich von 500-15 ng $\alpha_2$MS/ml Probe. Zur Spezifitätskontrolle wurde zusätzlich eine Probe mit gereinigtem $\alpha_2$MF (500 ng/ml) eingesetzt.

## TABELLE 2

| Quantifizierung von $\alpha_2$MF (Protease-komplexiertes $\alpha_2$M) im Rezeptor/Antikörper Festphasentest unter Verwendung von immobilisierten Streptokokkenrezeptoren (Stamm 1, DSM 5531) | | | |
|---|---|---|---|
| ng $\alpha_2$MF/ml Probe | A 414 nm | SD | VK (%) |
| 500 | 0.552 | 0.0141 | 2.5 |
| 250 | 0.490 | 0.0085 | 1.7 |
| 125 | 0.430 | 0.0119 | 2.8 |
| 60 | 0.366 | 0.0138 | 3.7 |
| 30 | 0.300 | 0.0145 | 4.8 |
| 15 | 0.245 | 0.0074 | 3.0 |
| 500 $\alpha_2$MS/ml Probe | 0.042 | 0.0052 | 12.3 |

Für die Quantifizierung von $\alpha_2$MF ergibt sich aus Tabelle 2 eine Standardkurve mit linearer Korrelation (r = 0.906) bei einem Konzentrationsbereich von 500-15 ng $\alpha_2$MF/ml Probe. Zur Spezifitätskontrolle wurde zusätzlich eine Probe mit gereinigtem $\alpha_2$MS (500 ng/ml) eingesetzt.

Im folgenden Versuch wurde unter Berücksichtigung der Standardkurven aus Tabelle 1 und 2 in verschiedenen humanen Normalplasmen der Gehalt an $\alpha_2$MS bzw. $\alpha_2$MF bestimmt.

Entsprechende Mikrotiterplatten wurden mit Streptokokken-Rezeptoren für $\alpha_2$MF beschichtet und unspezifische Bindungsstellen mit PBS Tween 1% BSA blockiert. Die Platten wurden anschließend mit drei verdünnten Plasmaproben, die unterschiedliche Mengen an $\alpha_2$MS bzw. $\alpha_2$MF enthielten, inkubiert. Pro Platte wurden 10 Verdünnungen angesetzt. Gebundenes $\alpha_2$M wurde anschließend über den monoklonalen Antikörper M2 und einen Enzym-markierten Detektionsantikörper in einer nachfolgenden Enzymreaktion quantifiziert. Die entsprechenden Konzentrationen an $\alpha_2$M in der Probe wurden anhand einer mitgeführten Standardkurve bestimmt. Ergebnisse für $\alpha_2$MS bzw. $\alpha_2$MF sind in Tabelle 3 und 4 aufgeführt. Angegeben sind die entsprechenden Mittelwerte der Absorption bei 414 nM, der Variationskoeffizient (VK) der entsprechenden Absorptionswerte und die mittels semilogarithmischer Regressionsanalyse ermittelten Konzentra-

tionen von $\alpha_2$MS bzw. $\alpha_2$MF.

Die Ergebnisse für $\alpha_2$MS sind in Tabelle 3 bzw. für $\alpha_2$MF in Tabelle 4 dargestellt.

TABELLE 3

| Quantifizierung von $\alpha_2$MS in humanen Plasmen mittels Rezeptor/Antikörper Festphasentest unter Verwendung von immobilisierten Streptokokkenrezeptoren (Stamm 2, DSM 5532 und Stamm 3, DSM 5533) | | | |
|---|---|---|---|
| Probe | A 414 nm | VK (%) | $\mu$g $\alpha_2$MS/ml Probe |
| A | 0.667 | 3.4 | 1200 |
| B | 0.525 | 4.5 | 300 |
| C | 0.255 | 5.8 | 75 |

TABELLE 4

Quantifizierung an $\alpha_2$MF (Protease-komplexiertes $\alpha_2$M) in humanen Normalplasmen mittels Rezeptor/Antikörper Festphasentest unter Verwendung von immobilisierten Streptokokkenrezeptoren (Stamm 1, DSM 5531)

| Probe | A 414 nm | VK (%) | $\mu$g $\alpha_2$MF/ml Probe |
|---|---|---|---|
| A | 0.487 | 3.1 | 125 |
| B | 0.334 | 2.2 | 31 |
| C | 0.256 | 4.8 | 3 |

Die Reproduzierbarkeit des Testsystems in der Serie wurde im folgenden Versuch ermittelt.

Auf entsprechend vorbschichteten Testplatten wurden drei Plasmaproben mit unterschiedlichen Gehalten an $\alpha_2$MS bzw. $\alpha_2$MF in Ansatz gebracht. Das über spezifische Rezeptoren gebundene $\alpha_2$M wurde mittels monoklonalem Antikörper M2 und einem anschließenden Detektionsantikörper in einer nachfolgenden Enzymreaktion nachgewiesen. Die Intra-Assay-Variation wurde für jedes Plasma in einem 16fachen Ansatz bestimmt. Ergebnisse für $\alpha_2$MS bzw. $\alpha_2$MF sind in Tabelle 5 und 6 wiedergegeben. Angegeben sind die Mittelwerte der Absorption bei 414 mM, die entsprechende Standardabweichung (SD) und der Intra-Assay-Variationskoeffizient (VK).

Ergebnisse für $\alpha_2$MS bzw. $\alpha_2$MF sind in Tabelle 5 und 6 wiedergegeben:

TABELLE 5

Intraassay Variationen bei der Quantifizierung von $\alpha_2$MS in humanen Normalplasmen mittels Rezeptor/Antikörper Festphasentest unter Verwendung von immobilisierten Streptokokkenrezeptoren (Stamm 2, DSM 5532 und Stamm 3, DSM 5533)

| Plasma-Probe | A 414 nm | SD | VK (%) Intraassay |
|---|---|---|---|
| A | 0.586 | 0.051 | 8.8 |
| B | 0.325 | 0.033 | 10.4 |
| C | 0.196 | 0.018 | 9.4 |

TABELLE 6

Intraassay Variationen bei der Quantifizierung von $\alpha_2$MF (Protease-komplexiertes $\alpha_2$M) in humanen Normalplasmen mittels Rezeptor/Antikörper Festphasentest unter Verwendung von immobilisierten Streptokokkenrezeptoren (Stamm 1, DSM 5531)

| Plasma-Probe | A 414 nm | SD | VK (%) Intraassay |
|---|---|---|---|
| A | 0.876 | 0.049 | 5.7 |
| B | 0.568 | 0.044 | 7.8 |
| C | 0.294 | 0.030 | 10.4 |

Im folgenden Versuch in vitro korrelierte der Gehalt an $\alpha_2$MF in einem Normalplasma mit der Zugabe von unterschiedlichen Mengen einer Peptidase (in Tab. 7 exemplarisch bovines Trypsin).

Einem humanen Normalplasma wurden verschiedene Mengen von bovinem Trypsin (0.100 nm/ml Plasma) zugegeben und 15 Minuten bei Raumtemperatur inkubiert. Anschließend wurde der Anteil an $\alpha_2$MS bzw. $\alpha_2$MF in den verschiedenen Plasmaproben mittels des oben beschriebenen Testsystems ermittelt. Die entsprechenden Ergebnisse sind in Tabelle 7 wiedergegeben.

Physiologischerweise vorkommende Serin-Proteasen wie z.B. Plasmin, Elastase, zeigten in diesem Testansatz vergleichbare Ergebnisse. Die Anwendung der hier entwickelten Bestimmungsmethode für $\alpha_2$MS und $\alpha_2$MF zur Beurteilung pathophysiologischer vorliegender Peptidase-Aktivität in Körperflüssigkeiten bzw. Gewebeexsudaten liegt daher nahe.

7

TABELLE 7

| Veränderung des Anteils von $\alpha_2$MS and $\alpha_2$MF in humanem Normalplasma nach Zugabe von unterschiedlichen Mengen bovinem Trypsin | | |
|---|---|---|
| | %-Anteil | |
| nmol Trypsin/ml Plasma | $\alpha_2$MS | $\alpha_2$MF |
| 0 | 100 | 0 |
| 0.1 | 100 | 1.2 |
| 1 | 98 | 9.2 |
| 10 | 94 | 16.4 |
| 20 | 82 | 30 |
| 40 | 22 | 95 |
| 100 | 0.3 | 100 |

Da bei den Bestimmungen in den beiden Testverfahren eine durchschnittliche Abweichung von 10.9 % bzw. 10.2 % vom Mittelwert ermittelt wurde, ergibt die Summe der Prozentangaben nicht genau 100. Wie aus Tabelle 7 jedoch hervorgeht, wird durch die Zugabe der Peptidase die Menge an detektiertem $\alpha_2$MS in der Probe reduziert, wohingegen die Menge an detektiertem $\alpha_2$MF zunimmt.

Beispiel 2

Im nachfolgenden wird die exemplarische Durchführung des Testsystems zur enzymatischen Quantifizierung einer mit $\alpha_2$M im Komplex vorliegenden Peptidase (humaner Leukozytenelastase) gezeigt.

| Aufbau eines Testprinzips zur Quantifizierung von mit $\alpha_2$M komplexierter Elastase | | |
|---|---|---|
| Schritt | Zeit | Substanzen |
| Beschichtung | über Nacht | z.B. Mikrotiterplatten F16 (High binding Cap.; Nunc GmbH, Wiesbaden) mit Streptokokkenlysat (*) 100 ug/ml (100 ul/well) |
| Blockierung | 1 h | PBS-TW. 1% BSA (2 mMol PMSF) (200 ul/well) |
| Proben-Inkubation | 1 h | Verdünnung in PBS-TW. 1 % BSA (100 ul/well) |
| Peptidase-Reaktion | variabel | z.B. Elastase-Substrat ($^+$) L-1335 (1 mg/ml) 1:20 verdünnt in Substratpuffer (0,5 M Tris pH 8,8) 50 ul/Vertiefung) bei 37° C inkubieren |
| Messung | | im Plattenphotometer bei 405 nm` |

(*) Für die Bestimmung wird das $\alpha_2$MF-Protease spezifische Streptokokkenlysat Stamm 1, DSM 5531 verwendet. Zur Kontrolle wurden die Proben parallel auf Testplatten inkubiert, die mit $\alpha_2$MS-spezifischen Lysa en (Stamm 2 und Stamm 3) getestet und entsprechende Extinktionen substrahiert.
($^+$) Zum Nachweis $\alpha_2$M-komplexierter humaner Leukozytenelastase wurde ein synthetisches elastasespezifisches Peptidsubstrat L-1335 der Firma Bachem eingesetzt.

TABELLE 8

Nachweis von $\alpha_2$ MF-Elastasekomplexen mittels eines kombinierten Rezeptor-vermittelten enzymatischen Festphasentestes
unter Verwendung von immobilisierten Streptokokkenrezeptoren
(Stamm 1, DSM 5531; Stamm 3, DSM 5533)

| ng Elastase/ml | A 405 nm |
|---|---|
| 0 | 0.008 |
| 1000 | 0.125 |
| 500 | 0.120 |
| 250 | 0.110 |
| 125 | 0.080 |
| 60 | 0.066 |
| 30 | 0.028 |
| 15 | 0.010 |

**Ansprüche**

1. Verfahren zur spezifischen Bestimmung der nativen Form oder der komplexierten Form von $\alpha_2$-Makroglobulin,
**dadurch gekennzeichnet,**
daß man die Lösung mit einem für die nachzuweisende $\alpha_2$-Makroglobulinform spezifischen immobilisierten Rezeptor und einem mit $\alpha_2$-Makroglobulin bindefähigen markierten Rezeptor inkubiert, wobei man als spezifische Rezeptoren diejenigen der Streptokokken-Spezies Stamm 2 (DSM 5532), Stamm 3 (DSM 5533) oder von Streptokokkus spp Stamm 1 (DSM 5531) verwendet, feste von flüssiger Phase trennt und nach Trennung der Phasen die Markierung in einer der beiden Phasen mißt.

2. Verfahren nach Anspruch 1,
**dadurch gekennzeichnet,**
daß man als mit der freien Form von $\alpha_2$-Makroglobulin spezifisch bindenden Rezeptor einen Rezeptor der Streptokokken-Spezies Stamm 2 (DSM 5532) oder Stamm 3 (DSM 5533) verwendet.

3. Verfahren nach Anspruch 1,
**dadurch gekennzeichnet,**
daß man als mit der komplexierten Form von $\alpha_2$-Makroglobulin-spezifisch bindefähigen Rezeptor den von Streptokokkus spp Stamm 1 (DSM 5531) gewonnenen verwendet.

4. Verfahren nach einem der vorhergehenden Ansprüche,
**dadurch gekennzeichnet,**
daß man als markierten, mit $\alpha_2$-Makroglobulin bindefähigen Antikörper einen von der Zellinie ECACC M2 89100605 produzierten monoklonalen Antikörper verwendet.

5. Verfahren nach einem der vorhergehenden Ansprüche,
**dadurch gekennzeichnet,**
daß man die komplexierte Form von $\alpha_2$-Makroglobulin bestimmt und anschließend zum Nachweis von Art und Konzentration des in dem $\alpha_2$-Makroglobulin gebundenen Enzyms ein für dieses Enzym spezifisches Substrat zugibt, das durch Einwirkung des zu bestimmenden Enzyms einen optisch nachweisbaren Rest bildet.

6. Verfahren nach Anspruch 5,

**dadurch gekennzeichnet,**
daß man als Substrat ein Protein mit einem Molekulargewicht von < 20 kDa verwendet.

Europäisches
Patentamt

**EUROPÄISCHER
RECHERCHENBERICHT**

Nummer der Anmeldung

**EP 90 12 0730**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,P | JOURNAL OF IMMUNOLOGICAL METHODS, Band 126, Nr. 1, Januar 1990, Seiten 103-108; C.W.E. JUSTUS et al.: "Quantification of free alpha2-macroglobulin and alpha2-macroglobulin-protease complexes by a novel ELISA system based on streptococcal alpha2-macroglobulin receptors" * Insgesamt * | 1-3 | G 01 N 33/68 G 01 N 33/573 G 01 N 33/566 |
| Y | IDEM | 5,6 | |
| Y | WO-A-8 402 778   (M. TEODORESCU et al.) * Zusammenfassung; Seite 4, Zeile 37 - Seite 5, Zeile 34 * | 5,6 | |
| A | CHEMICAL ABSTRACTS, Band 105, Nr. 5, 4. August 1986, Seite 519, Zusammenfassung Nr. 40320x, Columbus, Ohio, US; H.P. MUELLER et al.: "Binding of human alpha2-macroglobulin to streptococci of group A, B, C, and G", & RECENT ADV. STREPTOCOCCI STREPTOCOCCAL DIS., PROC. LANCEFIELD INT. SYMP. STREPTOCOCCI STREPTOCOCCAL DIS., 9th 1984 (Pub. 1985), 96-8 * Zusammenfassung * | 1-3 | |
| A | BIOLOGICAL ABSTRACTS, Band 72, Nr. 11, 1981, Zusammenfassung Nr. 74441, Biological Abstracts, Inc., Philadelphia, PA, US; A. BOUVET et al.: "Nutritionally deficient streptococci: Electron microscopic study of 14 strains isolated in bacterial endocarditis", & ANN. MICROBIOL. (PARIS), 131(2): 101-120. 1980 * Zusammenfassung * | 1-3 | RECHERCHIERTE SACHGEBIETE (Int. Cl.5) G 01 N |

Der vorliegende Recherchenbericht wurde für alle Patentansprüche erstellt

| Recherchenort | Abschlußdatum der Recherche | Prüfer |
|---|---|---|
| Den Haag | 11 Januar 91 | HITCHEN C.E. |